# FASCICULE DE BREVET EUROPEEN

(11) **EP 1 622 596 B1**
(45) Date de publication et mention de la délivrance du brevet: **14.10.2015**
(21) Numéro de dépôt: 04742644.0
(22) Date de dépôt: 05.05.2004
(51) Int. Cl.: A61K 9/70, A61L 15/00, A61K 38/42

(54) **IMPLANT INJECTABLE DE GLOBINE INSOLUBLE**
UNLÖSLICHES INJIZIERBARES GLOBIN-IMPLANTAT
INSOLUBLE GLOBIN INJECTABLE IMPLANT

(30) Priorité: 12.05.2003 FR 0305700
(43) Date de publication de la demande: 08.02.2006
(73) Titulaire: Khorionyx, 69890 La Tour de Salvagny (FR)
(72) Inventeur: TAYOT, Jean-Louis, c/o Khorionyx, 69890 La Tour de Salvagny (FR)
(74) Mandataire: Colombet, Alain André
(86) Numéro de dépôt international: PCT/FR2004/001082
(87) Numéro de publication internationale: WO 2004/100934

(56) Documents cités:
- EP-A- 0 134 729
- WO-A-98/19718
- WO-A-99/17786
- GB-A- 494 327
- US-A1- 2002 122 816
- DATABASE FSTA INTERNATIONAL FOOD INFORMATION SERVICE (IFIS), FRANkFURT-MAIN, DE; 1984, XP002295466 Database accession no. 84-4-11-S2297 cité dans la demande & AUTIO K. ET AL.: "CHEMICAL AND FUNCTIONAL PROPERTIES OF BLOOD GLOBIN PREPARED BY A NEW METHOD" JOURNAL OF FOOD SCIENCE, vol. 49, no. 3, 1984, pages 859-862,
- DATABASE FSTA INTERNATIONAL FOOD INFORMATION SERVICE (IFIS), FRANkFURT-MAIN, DE; 1985, XP002295467 Database accession no. 85-1-11-S0013 & AUTIO K. ET AL.: "PENETRATION STUDIES OF BLOOD GLOBIN GELS" JOURNAL OF FOOD SCIENCE, vol. 50, no. 3, 1985, pages 615-617,

## Description

La présente invention a pour but de fournir des préparations de globine utiles pour une administration à l'homme. Ces préparations peuvent se présenter, notamment, sous forme de pâtes injectables ou de matériaux solides implantables, ou d'implants.

On a déjà décrit de nombreuses applications médicales du collagène, que ce soit sous forme de pâtes, par exemple pour le comblement, de formulations fluides ou solides, comme des films ou des compresses, ou sous forme d'implants divers. En fait, seul le collagène animal est généralement utilisé.

La préparation de collagène humain, qui serait préférable au collagène animal, est envisageable à partir de tissus cutanés humains. Mais elle est rendue très difficile car le prélèvement de tissus humains à partir de cadavres pose des problèmes éthiques considérables et nécessite des tests coûteux pour éliminer les risques de transmission de maladies infectieuses, virales ou autres. La préparation de collagène humain à partir de placentas est coûteuse, complexe et difficile à organiser. La préparation de collagène humain par les méthodes modernes de recombinaison génétique ou de cultures cellulaires est aussi très coûteuse, ce qui gênera certainement le développement commercial de ce produit.

La globine est la protéine constitutive de l'hémoglobine qui, elle même, contient 4 chaînes peptidiques (2 chaînes α et 2 chaînes β) chacune associée à un hème. L'hème est constitué d'une structure tétrapyrole contenant 1 atome de fer chargé positivement. Il y a 4 hèmes par molécule, responsables de la coloration rouge de l'hémoglobine.

Les procédés de préparation de la globine sont connus depuis très longtemps et ont été développés dans le but d'application alimentaire ou pour la préparation de solutions pharmaceutiques injectables.

Contrairement à l'hémoglobine qui est parfaitement soluble à pH physiologique, la globine est remarquablement insoluble dans les mêmes conditions. Le caractère insoluble de la globine dans des conditions physiologiques a gêné jusqu'à présent le développement de ses applications pharmaceutiques. C'est pourquoi la plupart des essais ont cherché à préparer des dérivés solubles de la globine à pH physiologique, notamment par succinylation à l'aide de l'anhydride succinique ou par acétylation à l'aide de l'anhydride acétique, ou par hydrolyse des fonctions amides à pH alcalin, ce qui augmente la charge négative de la globine et diminue son pH isoélectrique.
Un produit injectable associant une préparation soluble de globine acide avec de l'insuline a été mis au point, breveté et commercialisé : REINER (1939) ; REINER et al. (1939). Il permet après injection, une délivrance progressive de l'insuline à partir de ce complexe : RABINOWITCH et al. (1947) ; BERG et al. (1953). La globine n'est pas l'élément actif, ni l'élément principal de ce produit.

La présente invention se propose de fournir de nouveaux matériaux et préparations injectables ou implantables dans l'organisme, dont la globine est l'élément actif principal et qui ne présentent pas les inconvénients des matériaux et formulations connus, par exemple de collagène ou autres.

L'invention a pour objet une préparation pâteuse ou solide de globine insoluble au pH physiologique, biocompatible, stérile et, de préférence, biodégradable, notamment sous forme de pâte injectable, de matériaux solides, par exemple de granules,de films, ou d'implants insolubles.

La présente invention se propose de conserver le caractère insoluble, naturel à pH neutre, de la globine, par exemple en récoltant par centrifugation un précipité protéique de globine formé par suspension de ce précipité dans un véhicule pharmaceutiquement acceptable, par exemple une solution aqueuse physiologique de type PBS, contenant 9g/l NaCl et tamponnée à pH neutre entre 6.8 et 7.5. La pâte ainsi formée est injectable après homogénéisation à l'aide d'une fine aiguille. Cette pâte peut être préparée en présence d'agents visqueux et lubrifiants comme des solutions de triglycérides, de polyéthylène-glycol, de hyaluronate, notamment de sodium, d'acide hyaluronique ou d'autres polysaccharides ou mucopolysaccharides ou de cellulose oxydée. Un tel additif facilite le passage du précipité pâteux à travers les plus fines aiguilles (diamètre 30g) et son injection comme implant intradermique.

L'originalité et l'intérêt de ce produit résident dans le fait qu'il s'agit d'une pâte protéique, parfaitement biocompatible avec les tissus environnants dans lesquels elle est injectée. Cette protéine n'a subi aucune altération ni modification chimique, elle est naturellement insoluble dès qu'elle se trouve dans un environnement physiologique. Une pâte protéique de globine humaine peut être utilisée chez l'homme pour combler des cavités, des rides, des cicatrices cutanées ou augmenter le volume de certains tissus (sphincters urinaires, digestifs, cordes vocales etc....). Cette pâte peut être utilisée pour combler des défauts de l'os ou du cartilage et pour en faciliter la cicatrisation. Des particules de globine insoluble peuvent aussi être utilisées en culture de cellules animales ou humaines. Les cellules de charge électrique négative s'attachent à la surface des particules de globine chargées positivement et se multiplient à leur surface. La dégradation progressive du support de globine, au contact des cellules qui le digèrent progressivement, peut fournir en plus un moyen de nutrition des cellules complémentaire ou alternatif aux milieux de culture liquides utilisés jusqu'ici.

Les applications de comblement permises par cette pâte de globine sont donc nombreuses et inattendues pour cette protéine.

La globine humaine homologue est préférable et permet d'éviter toute réaction immunologique du patient à traiter, pendant ou après l'injection. Ce produit représente donc un avantage important par rapport au collagène qui jusqu'à présent est préparé à partir de peaux d'animaux (veau, porc, etc....) et qui nécessite un certain nombre de précautions et conditions pour éviter les réactions immunologiques chez les patients.
- Nécessité de tester chaque patient pour une éventuelle allergie au collagène animal
- Impossibilité de traiter les personnes allergiques

La globine reste cependant soluble à des pH acides ou basiques et dans ces conditions peut être filtrée stérilement sur des membranes poreuses. Pour des concentrations appropriées de 20 à 300 mg/ml, de telles solutions peuvent être traitées comme des solutions de protéines et permettent de réaliser des produits tels que : éponges, films, granules, en utilisant ou combinant les techniques de séchage, lyophilisation, réticulation, précipitation. Quelques exemples sont développés ci-dessous.

La globine est facile à purifier à partir de globules rouges provenant de sang animal ou humain. Les globules rouges humains sont disponibles en quantité suffisante à partir des dons périmés restant en stock dans les centres de transfusion sanguine et pour lesquels tous les tests préalables sanitaires ont été réalisés au moment du prélèvement. La préparation de globine insoluble, injectable ou d'autres biomatériaux à base de globine représente donc de nouvelles applications biomédicales permettant de valoriser le sang non utilisé ou les dons de sang périmé et d'éviter ou de diminuer leur destruction.

La mise en oeuvre de l'invention est possible aussi à partir d'un prélèvement d'échantillon de sang du patient à traiter d'environ 5 à 100 ml, et sa transformation en globine autologue avec les mêmes méthodes que pour de grands volumes, puis l'injection de la pâte obtenue pour la correction des rides du même patient ou d'autres applications telles que la cicatrisation des plaies chroniques. Le nombre de seringues préparées à partir d'un prélèvement du patient peut être important et permettre le traitement du patient pendant plusieurs années.

De même le placenta humain qui est expulsé après l'accouchement contient du sang qui est généralement détruit par incinération, mais qui peut servir aussi à l'invention.

Les poches de sang de donneurs sont contrôlées officiellement par les organismes de transfusion sanguine, grâce aux examens biochimiques, bactériologiques, sérologiques et tests de screening des différents virus et d'autres agents infectieux. Dans le cas du sang placentaire, il serait évidemment nécessaire de réaliser les mêmes examens sur des échantillons de sang du cordon ombilical ou de la mère avant de pouvoir collecter, conserver et extraire le sang de cette matière première. Pour le sang autologue, les tests à effectuer peuvent être simplifiés.

La réalisation de l'invention nécessite d'abord de recueillir et purifier les globules rouges dans ces échantillons de sang, ou liquides sanguins, par des opérations simples et déjà connues. Les globules rouges sont récupérés par centrifugation à basse vitesse. Le surnageant plasmatique est séparé et remplacé par un liquide salin physiologique de type PBS ; contenant 9g/l de NaCl et tamponné à pH neutre.

Après plusieurs lavages (3 à 5), la suspension de globules rouges est ainsi débarrassée des protéines du plasma. Le culot de globules rouges purifiés est additionné de 1 ou 2 volumes d'eau distillée pour réaliser un choc osmotique qui entraîne la lyse des membranes des globules rouges et libère l'hémoglobine en solution concentrée et purifiée. Une étape de centrifugation à haute vitesse (10 à 20 000 t/mn) permet d'éliminer les débris membranaires et cellulaires dans le culot. Une étape finale de filtration du surnageant sur membrane de porosité de 0.2 micron permet de préparer une solution d'hémoglobine purifiée et stérile, dépourvue de particules et dérivés d'origine tissulaire, cellulaire ou membranaire.

Le clivage Hème-Globine à pH acide a été décrit en présence d'alcool par SCHULZ dès 1898. ANSON et MIRSKY en 1930 puis ROSSI-FANELLI et coll. en 1958 utilisent l'acétone en présence d'acide à 0°C. TEALE (1957) préfère l'utilisation de la méthyl-éthyl cétone à la place de l'acétone. AUTIO et coll. (1984) séparent la globine à pH acide grâce à l'absorption et la précipitation de l'hème avec la carboxymethylcellulose soluble. La globine ainsi préparée est soluble à pH acide ou alcalin mais devient insoluble dès que le pH de la solution aqueuse est neutralisé entre pH 6 et 8.

Des essais de solubilisation à pH neutre ont été réalisés par STRUMIA et coll. en 1951 et 1952 par un traitement alcalin prolongé qui entraîne une déamidation partielle de la globine au niveau des résidus asparagine et glutamine transformés respectivement en acide aspartique et acide glutamique (VARS 1952). D'autres essais de solubilisation ont été réalisés par VOLCKMANN en 1988 par succinylation.

Le caractère insoluble en milieu physiologique explique la persistance de la globine après implantation tissulaire, ce qui la rend aussi résistante à une dégradation enzymatique,surtout si la quantité injectée est importante, ce qui est le cas dans les applications de comblement ou d'augmentation tissulaire.

Au contraire, la plupart des autres protéines ne sont précipitables que par des concentrations élevées de sels ou d'alcool, ce qui rendrait leurs précipités non biocompatibles et non utilisables pour des injections intra tissulaires. De plus, de tels implants disparaîtraient très vite par diffusion des agents précipitants et dissolution progressive du précipité au contact du milieu physiologique des tissus.

La vérification de l'intérêt de l'invention peut être facilement réalisée à partir d'une préparation de globine de lapin. La pâte de globine précipitée, physiologique, ainsi préparée peut être injectée par voie sous-cutanée en différents endroits sur le dos ou la paroi du lapin. Il est facile de vérifier l'innocuité par l'absence d'érythème localement. La persistance du produit sous la peau peut être observée par palpation en fonction du temps. L'absence de pouvoir antigénique du produit peut être vérifiée par immunisation de lapins avec ou sans adjuvant de Freund par voie sous cutanée et intra musculaire. Les prélèvements de sang effectués après l'immunisation permettent de vérifier l'absence d'anticorps anti-globine ou anti-hémoglobine par les tests habituels de contrôle.

### Exemples de réalisation de produits selon l'invention.

### Exemple 1 : Préparation de globine de lapin

Cinq lapins anesthésiés sont saignés par ponction cardiaque. Le sang est récupéré en présence d'héparine ou en présence de citrate de sodium pour éviter sa coagulation. On obtient ainsi 210 ml de sang qui sont centrifugés pendant 30 minutes à 2500 t/mn. Le surnageant contenant le plasma est prélevé avec une pipette et le culot est lavé 5 fois par 3 volumes de tampon PBS, contenant 9 g/l NaCl et tamponné à pH 7.2. Le culot final, lavé, est additionné, sous agitation, d'un volume égal d'eau distillée pour lyser les hématies. La suspension finale est centrifugée à 12 000t/mn pour éliminer des débris cellulaires et membranaires. Le surnageant est filtré sur membrane d'acétate de cellulose de porosité 0.22 micron. On obtient 82 ml contenant 97 g/l d'hémoglobine.

L'hémoglobine est transformée en globine selon la technique décrite par TAYOT et VERON (1983). Cette solution d'hémoglobine est versée sous agitation dans 275 ml d'éthanol 96% contenant 1 ml d'HCL concentré. Le pH est ajusté à 3. La concentration finale est de 74% d'éthanol et de 22g/l d'hémoglobine à pH acide. On ajoute 3 g de charbon actif L4S de la marque CECA sous agitation vigoureuse pendant 15 heures à 4° C.

La suspension est centrifugée à 15 000t/mn pendant 30 minutes pour éliminer le charbon sous forme de culot. Le surnageant contenant la globine acide décolorée est filtré sur une série de membranes poreuses jusqu'à la porosité la plus faible (0.2 micron) pour éliminer les particules fines de charbon. Le filtrat est dilué par un volume égal d'eau distillée, le pH est ajusté à 7.4 par addition de NaOH et la globine précipite massivement. Après 15 heures, le précipité de globine est récupéré par centrifugation, puis lavé 2 fois par 3 volumes de solution physiologique PBS, contenant 9g/l NaCl et tamponnée à pH 7.4. On récolte 58,2 g de précipité de globine à pH 7.4. Le précipité est homogénéisé par transferts successifs entre 2 seringues de volume 5 ml reliées par un connecteur de diamètre intérieur de 1 à 0,2 mm, en appuyant successivement sur le piston de chaque seringue pour faire passer le précipité dans l'autre seringue.

En final, le précipité homogénéisé est réparti dans des seringues de 1 ml. Il est possible d'expulser la pâte de globine précipitée à partir de la seringue, à travers des aiguilles fines de diamètre 24 ou 27g. La concentration de globine dans la pâte peut être ajustée à des valeurs comprises entre 30 et 150 mg/g.

### Exemple 2 : Préparation de globine humaine

200 ml de sang humain périmé, prélevé sur citrate de sodium sont centrifugés pendant 30 mn à 2500 t/mn. Le surnageant contenant le plasma est prélevé avec une pipette en aspirant aussi la couche cellulaire blanchâtre superficielle correspondant aux leucocytes. Le culot de globules rouges est lavé 5 fois avec 3 volumes de solution physiologique PBS, contenant 9 g/l NaCl et tamponné à pH 7.2, par des centrifugations successives. Le culot final est additionné de 2 volumes d'eau distillée pour lyser les hématies. La suspension hémolysée est clarifiée par centrifugation pendant 30 mn à 12 000t/mn et filtrée sur membrane de porosité 0.2 micron. On obtient 210 ml contenant 52 g/l d'hémoglobine qui sont conservés à 4° C.

Un volume égal de 210 ml d'HCl 0.1 N à 4°C est ajouté puis l'ensemble est versé dans 4 1 d'acétone, contenant 40 ml d'HCl 1 N. La suspension est agitée vigoureusement et laissée au repos pendant 1 heure à température ambiante, sous une hotte chimique. L'hème dissout dans l'acétone est éliminé par filtration sur toile poreuse et le précipité de globine est récupéré, lavé en acétone acide et séché sous courant d'air.

En variante, divers acides minéraux (sulfurique, phosphorique...)ou carboxyliques, tels que l'acide acétique, l'acide oxalique, ou l'acide citrique par exemple, peuvent être utilisés à la place de l'acide chlorhydrique pour acidifier la solution d'hémoglobine avant sa décoloration.

Une autre variante de ce procédé consiste à précipiter la solution acide d'hémoglobine avant sa décoloration. La précipitation peut être réalisée par addition de NaCl à une concentration de 40 à 60 g/l. Le précipité d'hémoglobine acide est ensuite décoloré par suspension dans un volume suffisant d'éthanol ou/et d'acétone. Le pigment passe en solution dans l'éthanol ou/et l'acétone ; la globine reste sous forme précipitée et peut être récoltée par filtration sur toile poreuse. Grâce à l'élimination de toute phase aqueuse, cette variante permet de réduire le volume nécessaire d'éthanol ou/et d'acétone d'un facteur au moins égal à 5.

La globine est remise en solution aqueuse à pH compris entre 2 et 3. La solution aqueuse de globine acide est filtrée stérilement sur membrane de porosité 0.2 micron, puis précipitée par neutralisation du pH par addition de NaOH jusqu'à pH 7.4. Des seringues de pâte de globine, précipitée à pH neutre, peuvent être préparées comme dans l'exemple précédent. L'opération de neutralisation de la solution de globine acide peut s'effectuer en ajoutant du hyaluronate de sodium à pH alcalin. Dans ce cas il se forme une pâte de globine insoluble complexée et imprégnée par le hyaluronate, apportant une fonction lubrifiante qui améliore le caractère injectable à travers de très fines aiguilles (diamètre 30g).

### Exemple 3 : Autre préparation de globine humaine

On réalise le procédé de l'exemple 1 à partir de culot globulaire contrôlé et périmé obtenu auprès d'un centre de transfusion sanguine. On obtient des seringues contenant une pâte de globine humaine, précipitée, biocompatible et implantable par injection.

### Exemple 4 : Préparation de globine humaine ayant subi un traitement alcalin par la soude 0.1 ou 1 M pendant 1 heure à 20 °C

On réalise le procédé de l'exemple 1 ou 2 jusqu'à l'obtention du précipité de globine à pH 7.4, avant lavages. Ce précipité est dissout, à nouveau, dans 3 volumes de soude NaOH 0.1 M à 1M à 20°C pendant 1 heure, sous agitation.

La solution est ensuite neutralisée par addition d'un volume égal d'HCl de la même molarité et le pH de la suspension est ajusté entre 7 et 7.4. Le précipité de globine est alors récolté par centrifugation, puis lavé en solution physiologique PBS comme dans les exemples précédents. La pâte de globine précipitée, pouvant être additionnée d'acide hyaluronique, ou d'autres produits visqueux et lubrifiants biocompatibles : triglycérides, polyéthylène glycol, cellulose oxydée, chitosane,etc... est répartie en seringues et on vérifie à nouveau le caractère injectable du produit obtenu à travers de très fines aiguilles pour usage intradermique. Ce traitement alcalin de la globine permet d'améliorer les garanties de sécurité sanitaire du produit sans modifier de manière significative le caractère insoluble de la globine à pH neutre.

### Exemple 5 : Préparation d'une pâte de globine précipitée et réticulée par le glutaraldéhyde.

Ce traitement est possible pour augmenter le temps de résorption de l'implant. Le précipité final de globine est mis en suspension à 2% dans du PBS. Le glutaraldéhyde est ajouté sous agitation à une concentration de 1 mg/g de précipité. Après incubation de 1 heure à 20° C, le précipité de globine est lavé et mis en seringue comme dans les exemples précédents.

D'autres agents de réticulation comme des dialdéhydes ou des polyaldéhydes peuvent être utilisés, notamment des polysaccharides oxydés par l'acide périodique tels que le dextran oxydé, l'amidon oxydé, ou l'acide hyaluronique oxydé.

### Exemple 6 : Préparation de seringues de pâte de globine précipitée stérile

Pour préparer des seringues stériles, il est nécessaire de travailler dans des conditions aseptiques aussitôt la filtration stérilisante de la solution de globine acide sur membrane de porosité 0.2 micron. Ceci peut se faire sous hotte à flux laminaire dans une zone stérile de classe 100 ou 1000, ou avec une enceinte stérile, accessible de l'extérieur par des gants souples de latex. Les opérations de précipitation, de décantation, ou de centrifugation du précipité doivent s'effectuer dans des pots stérilisés et emballés sous film protecteur.

Une autre méthode consiste à répartir une solution acide de globine soluble, filtrée stérilement dans une première seringue et une deuxième solution alcaline, filtrée stérilement dans une deuxième seringue. Le pH de chaque seringue est ajusté de manière que leur mélange ultérieur soit à pH neutre. La connexion de ces deux seringues, grâce à un connecteur stérile permet de réaliser un mélange stérile, homogène, de pH neutre, par des transferts successifs d'une seringue dans l'autre. Un précipité stérile est obtenu par précipitation spontanée de la globine. La suspension obtenue peut être concentrée par extrusion à travers de fines aiguilles qui ne laissent passer que la phase aqueuse. L'addition éventuelle de hyaluronate de sodium ou de tout autre agent visqueux et lubrifiant dans la seringue de globine concentrée permet de l'incorporer dans la globine finale. En variante, il est possible d'incorporer aussi un agent de réticulation, au moment du mélange des deux seringues initiales, pour allonger le temps de résorption in vivo de la globine.

### Exemple 7 : Stérilisation finale des seringues de pâte de globine précipitée

Une stérilisation des seringues préparées selon l'un quelconque des exemples précédents peut être réalisée par irradiation à une dose comprise entre 5 et 30 kilogray. Les diverses préparations de globine sont insolubles avant comme après leur stérilisation par irradiation. Dans les deux cas, la globine insoluble à pH neutre devient soluble si l'on opère une acidification à pH 3 par toute solution aqueuse acide.

### Exemple 8 : Réalisation d'un gel ou d'un film insoluble à partir de globine soluble non modifiée.

Une solution de globine soluble est réalisée en dissolvant la poudre acétonique de globine à pH 3, à une concentration de 20 à 120 mg/ml en solution aqueuse. Cette solution est filtrée stérilement sur membrane de porosité 0,2µ, puis ajustée à pH 5 par addition de soude stérile NaOH 1 N sous agitation.

Le mélange est additionné d'amidon oxydé à pH 3,5, ou d'un autre aldéhyde, ou polyaldéhyde réticulant, contenant au moins 5 atomes de carbone par molécule, à une concentration de 0,5% sous agitation pendant 5 mn. Le mélange stérile est coulé sur une surface plane pour obtenir une épaisseur de 1 à 3 mm de liquide, à une température de 20 à 37°C, sous flux laminaire.

Le produit liquide se gélifie progressivement grâce à la réticulation des chaînes de globine induite par l'amidon oxydé, puis se déshydrate sous le courant d'air, si on souhaite obtenir un film.

Le film final d'épaisseur comprise entre 20 et 200µ, selon la concentration initiale de matière, peut être stérilisé par irradiation béta ou gamma ou par l'oxyde d'éthylène. Des agents de filmage bien connus peuvent être ajoutés tels que le collagène, la gélatine, l'acide hyaluronique, la cellulose oxydée ou d'autres polysaccharides ou mucopolysaccharides, le polyéthylène-glycol, le glycérol etc. Un tel additif permet de donner de la souplesse et/ou de la résistance au film. Un tel film peut être utilisé seul pour protéger une plaie cutanée ou chirurgicale, favoriser la cicatrisation, ou peut être associé à diverses prothèses (prothèses vasculaires, treillis de renfort, matrices poreuses) pour les rendre imperméables, ou améliorer leur biocompatibilité, ou conférer des propriétés anti-adhérences ou un caractère adhésif, ou accélérer la colonisation cellulaire de ces prothèses, selon des techniques connues et déjà employées avec d'autres produits.

Dans une variante, il est possible de réaliser un film à partir de globine soluble à pH 5 comme précédemment, mais sans introduire d'agent de réticulation. La réticulation finale du film séché est réalisée par l'irradiation finale qui crée des liens covalents entre les chaînes de globine. Un tel film peut être collé ensuite sur les tissus à l'aide d'adhésifs biologiquement compatibles, réactifs avec les groupements aminés de la globine. De préférence les polyaldéhydes obtenus par oxydation périodique de polysaccharides sont utilisables. A titre d'exemple le dextrane oxydé ou l'amidon oxydé sont préférés.

### Exemple 9: Applications biomédicales des particules de globine insolubles comme support de cultures cellulaires.

La pâte de précipité de globine à pH neutre, stérile, obtenue comme dans l'un quelconque des exemples précédents, est incubée par exemple avec du milieu DMEM pour cultures cellulaires, à une température voisine de 37°C. On obtient une suspension dans laquelle les cellules sont introduites à une densité de 10000 à 100000 cellules/ml. Après agitation de 30 minutes et décantation de 1 heure à 15 heures, les cellules s'attachent aux particules de globine et se multiplient à leur surface pendant la durée de la culture qui peut être de 3 à 12 jours. Le milieu de culture cellulaire est choisi en fonction du type cellulaire selon les connaissances actuellement publiées.

En fin de multiplication, la suspension cellulaire fixée aux particules de globine, peut être concentrée par décantation spontanée. La pâte cellulaire obtenue peut être mise en seringues et injectée comme implant cellularisé biocompatible pour des applications thérapeutiques diverses aujourd'hui connues.

La culture de fibroblastes cutanés, selon cette méthode, peut permettre la préparation d'implants cellularisés pour des applications de cicatrisation cutanée ou de comblement de tissus conjonctifs.

La culture de chondrocytes, selon cette méthode, peut permettre la préparation d'implants cellularisés pour des applications de comblement et cicatrisation de plaies superficielles du cartilage.

La culture d'ostéoblastes, selon cette méthode, peut permettre la préparation d'implants cellularisés pour des applications de comblement et cicatrisation de fractures ou pertes de substance osseuse.

De la même manière des cellules souches, notamment d'origine embryonnaire, ou de sang de cordon ombilical, ou de moelle osseuse, ou isolées à partir de différents tissus adultes, peuvent être cultivées sur ces particules de globine et remplir les fonctions recherchées après injection ou implantation de la pâte cellularisée.

Pour des applications biomédicales comme la fabrication de virus ou des vaccins dérivés, dans lesquelles les cellules peuvent être séparées, après culture, de leur support de globine, les méthodes traditionnelles de trypsination peuvent être employées. Les particules non dégradées de globine décantent spontanément au fond du flacon et peuvent être séparées des cellules par décantation.

Dans certaines variantes, il est possible de remplacer les particules de globine par des films contenant la globine insoluble. La culture des cellules peut alors s'effectuer par circulation continue du milieu de culture au contact de ces films plans, comme pour toutes cultures de cellules sur membranes ou films aujourd'hui connues. Cette méthode permet de réaliser également des films cellularisés qui peuvent être implantés pour des applications médicales spécifiques.

### Exemple 10: Applications médicales des implants de globine insoluble injectable.

Les préparations selon l'invention, et notamment les seringues de globine insoluble préparées selon l'un quelconque des exemples 1 à 7 peuvent être utilisées dans les applications suivantes non limitatives :
- Comblement des rides et défauts cutanés
- Comblement des tissus conjonctifs ou sphincters pour des applications en urologie : reflux vésico-urétéral de l'enfant, incontinence d'effort de la femme ; en O.R.L. : correction de volume des cordes vocales.
- Bouchon hémostatique pour les plaies artérielles percutanées.
- Cicatrisation cutanée, par utilisation de la pâte de globine seule ou en association avec d'autres produits de cicatrisation ou facteurs de croissance.
- Cicatrisation du cartilage ou de l'os, par utilisation de la globine seule ou en association avec d'autres produits de cicatrisation : phosphate de calcium, carbonate de calcium, hydroxyapatite, facteurs de croissance de type BMP.
- Association à des antibiotiques pour inhiber le développement bactérien, pendant la période de colonisation et dégradation de l'implant.

L'invention a également pour objet les procédés de traitement du corps humain ou animal comprenant au moins une étape d'administration d'une quantité thérapeutiquement efficace d'un préparation injectable ou implantable selon l'invention à un patient qui en présente le besoin.

Ces procédés comprennent notamment les administrations correspondant aux applications précitées, par les voies parentérales ou chirurgicales d'injection ou d'implantation, ou par voie cutanée.

### Bibliographie

ANSON M.L. - MIRSKY A.E. (1930) Protein Coagulation and its reversal. The preparation of insoluble globin, soluble globin and heme. J. Gen. Physiol. 13, 469-476
AUTIO K - KIESVAARA M. - MALKKI Y. - KANKU S. (1984) Chemical and functional properties of blood globin prepared by a new method Journal of Food Science 49, 859-862
BERG J.W. - ORTMEYER D.W. - OTT D.L. - JACKSON R.L. (1953) Comparison of Globin Insulin and NPH Insulin Diabetes, 2, 5, p.365-369
RABINOWITCH I.M. - FOWLER A.F. - BENSLEY E.H. - GORDON A.L.-MOUNTFORD M. (1947) Globin Insulin The Canadian Medical Association J., 56, 6, p.595-605
REINER L. (1939)
   Insulin preparation
   US Patent # 2161198
REINER L. - SEARLE D.S. - LANG E.H. (1939) Insulin preparations with prolonged activity I. Globin Insulin Proc. Soc. Exp. Biol. Med. 40, p.71
ROSSI-FANELLI A. -ANTONINI E. -CAPUTO A. (1958) Studies on the structure of haemoglobin I-Physicochemical properties of human globin Biochem. Biophys. Acta 30, 608-615
SCHULZ F.N. (1898) Der Eiweisskörper des hämoglobins Ztsch. F. physiol. chem. 24, 449-460
STRUMIA M.M. - SAMPLE A.B. - MAWR B. (1951) Modified globin I-Method for preparation from human erythrocytes. J. Lab.and Clin.Med. 37, 959-968
STRUMIA M.M. - Mc GRAW J.J. - SAMPLE A.B. - MAWR B. (1952) Modified globin IV- Some of the physiological properties of modified human globin J. Lab.and Clin. Med. 40, 2, 211-222
TAYOT J.L. - VERON J.L. (1983)
   Brevet Institut Mérieux : FR 8311324
   Process for preparing globin from haemoglobin and globin obtained by this process.
   US Patent 4543209 (1985)
TEALE F.W.J. (1957) Cleavage of the haem-protein link by acid methyl-ethyl keton Biochem. Biophys. Acta 26, 437
VARS H.M. -BOXER G.E. -MAWR B. (1952) Modified Globin II- Chemical changes in human globin by alkaline modification J. Lab.and Clin. Med. 39, 5, 743-751
VOLCKMANN H. (1988) Essais de développement d'un substitut plasmatique d'origine placentaire Thèse d'ingénieur CNAM - Lyon

## Revendications

1. Préparation solide ou pâteuse injectable ou implantable dans l'organisme humain et animal, comportant, à titre de composant actif principal, de la globine insoluble au pH physiologique, biocompatible et stérile, pour utilisation comme matériau de comblement tissulaire.

2. Préparation pour utilisation selon la revendication 1, pour le comblement de cavités, rides ou cicatrices cutanées et cavités et fractures de l'os ou du cartilage.

3. Préparation solide ou pâteuse injectable ou implantable dans l'organisme humain et animal, comportant, à titre de composant actif principal, de la globine insoluble au pH physiologique, biocompatible et stérile, pour utilisation pour l'augmentation de volume de tissus.

4. Préparation pour utilisation selon la revendication 3, **caractérisée en ce qu'**elle est prévue pour l'augmentation de sphincters, notamment urinaire ou digestif ou de cordes vocales.

5. Préparation solide ou pâteuse injectable ou implantable dans l'organisme humain et animal, comportant, à titre de composant actif principal, de la globine insoluble au pH physiologique, biocompatible et stérile, sous forme de film et/ou de compresse, pour utilisation pour la protection et/ou la séparation de plaies ou cicatrices externes ou internes, chirurgicales ou non.

6. Préparation solide ou pâteuse injectable ou implantable dans l'organisme humain et animal, comportant, à titre de composant actif principal, de la globine insoluble au pH physiologique, biocompatible et stérile, pour utilisation pour former un bouchon hémostatique pour plaies artérielles percutanées, ou une pâte de cicatrisation cutanée, un matériau de cicatrisation du cartilage ou de l'os.

7. Préparation pour utilisation selon l'une quelconque des revendications précédentes, **caractérisée en ce que** la globine est une globine d'origine humaine.

8. Préparation pour utilisation selon l'une quelconque des revendications précédentes, **caractérisée en ce que** la globine, dans la préparation, est à l'état précipité.

9. Préparation pour utilisation selon l'une quelconque des revendications précédentes, **caractérisée en ce qu'**elle comporte de la globine homogénéisée.

10. Préparation pour utilisation selon la revendication 9, **caractérisée en ce que** la globine se présente sous forme d'une pâte homogénéisée, injectable.

11. Préparation pour utilisation selon l'une des revendications 9 et 10, **caractérisée en ce que** la pâte homogénéisée est injectable à travers une aiguille hypodermique.

12. Préparation pour utilisation selon l'une des revendications 9 à 11, **caractérisée en ce que** la concentration de globine dans la préparation injectable est comprise entre 30 et 150 mg/g

13. Préparation pour utilisation selon l'une des revendications 9 à 12, **caractérisée en ce que** le pH de la préparation est compris entre 6 et 8.

14. Préparation pour utilisation selon l'une des revendications précédentes, **caractérisée en ce que** la globine est en suspension.

15. Préparation pour utilisation selon l'une des revendications 1 à 8, **caractérisée en ce que** la globine est présente dans la préparation sous forme d'un gel.

16. Préparation pour utilisation selon l'une quelconque des revendications précédentes, **caractérisée en ce qu'**elle comprend, en outre, un agent lubrifiant.

17. Préparation pour utilisation selon la revendication 16, **caractérisée en ce que** cet agent lubrifiant est choisi parmi des solutions de triglycérides, de polyéthylène glycol, de hyaluronate, d'acide hyaluronique, de cellulose oxydée, ou de polysaccharides ou de mucopolysaccharides.

18. Préparation pour utilisation selon l'une quelconque des revendications précédentes, **caractérisée en ce que** la préparation comporte un agent réticulant.

19. Préparation pour utilisation selon la revendication 18, **caractérisée en ce que** l'agent réticulant est choisi parmi le glutaraldéhyde, les dialdéhydes et les polyaldéhydes, notamment les polysaccharides oxydés par l'acide périodique, y compris le dextran oxydé, l'amidon oxydé ou l'acide hyaluronique oxydé.

20. Préparation pour utilisation selon l'une des revendications 1 à 8, **caractérisée en ce qu'**elle comporte ou est constituée par un film de globine, la préparation pouvant contenir optionnellement, un agent de filmage notamment tel que collagène, gélatine, acide hyaluronique, cellulose oxydée, polyéthylène glycol, glycérol.

21. Préparation pour utilisation selon la revendication 20, **caractérisée en ce que** le film a été obtenu par déshydratation d'un gel ou d'une solution.

22. Préparation pour utilisation selon l'une des revendications 1 à 8, **caractérisée en ce qu'**elle est réalisée sous forme d'un implant solide.

23. Préparation pour utilisation selon l'une des revendications 20 à 22, **caractérisée en ce qu'**elle est réticulée.

24. Préparation pour utilisation selon la revendication 23, **caractérisée en ce qu'**elle est réticulée par adjonction d'un agent réticulant et/ou par une irradiation.

25. Préparation pour utilisation selon l'une quelconque des revendications précédentes, **caractérisée en ce qu'**elle contient, en outre, l'un au moins des principes actifs suivants : produit de cicatrisation, facteur de croissance, antibiotique.

26. Préparation pour utilisation selon l'une quelconque des revendications précédentes, **caractérisée en ce qu'**elle contient des cellules, notamment des cellules cultivées en utilisant la globine de la préparation comme support de culture, avant injection ou implantation, cellules pouvant être notamment des fibroblastes cutanées, des chondrocytes, des ostéoblastes, ou des cellules souches.

## Patentansprüche

1. Feste oder pastöse, in den humanen oder tierischen Organismus injizierbare oder implantierbare Zusammensetzung, umfassend, als wesentliche Aktivkomponente, biokompatibles und steriles, bei physiologischem pH unlösliches Globin, für die Verwendung als Gewebefüllmaterial.

2. Zusammensetzung zur Verwendung gemäß Anspruch 1, für die Auffüllung von Vertiefungen, Falten oder Narben der Haut und Hohlräumen und Frakturen des Knochens oder Knorpels.

3. Feste oder pastöse, in den humanen oder tierischen Organismus injizierbare oder implantierbare Zusammensetzung, umfassend, als wichtigste Aktivkomponente, biokompatibles und steriles, bei physiologischem pH unlösliches Globin, für die Verwendung zur Erhöhung des Gewebevolumens.

4. Zusammensetzung zur Verwendung gemäß Anspruch 3, **gekennzeichnet dadurch, dass** sie zur Schließmuskelverstärkung vorgesehen ist, insbesondere des Harn-, oder Verdauungsschließmuskels oder der Stimmbänder.

5. Feste oder pastöse, in den humanen oder tierischen Organismus injizierbare oder implantierbare Zusammensetzung, umfassend, als wichtigste Aktivkomponente, biokompatibles und steriles, bei physiologischem pH unlösliches Globin, in Form eines Films und/oder einer Kompresse, für die Verwendung zum Schutz und/oder Abtrennung von externen oder internen, chirurgischen oder nicht-chirurgischen Wunden oder Narben.

6. Feste oder pastöse, in den humanen oder tierischen Organismus injizierbare oder implantierbare Zusammensetzung, umfassend, als wichtigste Aktivkomponente, biokompatibles und steriles, bei physiologischem pH unlösliches Globin, für die Verwendung zur Bildung eines blutstillenden Pfropfens für perkutane, arterielle Wunden, oder einer Hautwundheilpaste, eines Knorpel- oder Knochenwundheilmaterials.

7. Zusammensetzung zur Verwendung gemäß einem der vorangehenden Ansprüche, **gekennzeichnet dadurch, dass** das Globin ein vom Menschen stammendes Globin ist.

8. Zusammensetzung zur Verwendung gemäß einem der vorangehenden Ansprüche, **gekennzeichnet dadurch, dass** das Globin in der Zusammensetzung ein Präzipitat ist.

9. Zusammensetzung zur Verwendung gemäß einem der vorangehenden Ansprüche, **gekennzeichnet dadurch, dass** sie homogenisiertes Globin umfasst.

10. Zusammensetzung zur Verwendung gemäß Anspruch 9, **gekennzeichnet dadurch, dass** das Globin in Form einer homogenisierten, injizierbaren Paste vorliegt.

11. Zusammensetzung zur Verwendung gemäß einem der Ansprüche 9 und 10, **gekennzeichnet dadurch, dass** die homogenisierte Paste durch eine hypodermale Kanüle injizierbar ist.

12. Zusammensetzung zur Verwendung gemäß einem der Ansprüche 9 bis 11, **gekennzeichnet dadurch, dass** die Konzentration an Globin in der injizierbaren Zusammensetzung zwischen 30 und 150 mg/g beträgt.

13. Zusammensetzung zur Verwendung gemäß einem der Ansprüche 9 bis 12, **gekennzeichnet dadurch, dass** der pH der Zusammensetzung zwischen 6 und 8 beträgt.

14. Zusammensetzung zur Verwendung gemäß einem der vorangehenden Ansprüche, **gekennzeichnet dadurch, dass** das Globin suspendiert ist.

15. Zusammensetzung zur Verwendung gemäß einem der Ansprüche 1 bis 8, **gekennzeichnet dadurch, dass** das Globin in Form eines Gels in der Zusammensetzung vorliegt.

16. Zusammensetzung zur Verwendung gemäß einem der vorangehenden Ansprüche, **gekennzeichnet dadurch, dass** sie, unter anderem, einen Schmierstoff enthält.

17. Zusammensetzung zur Verwendung gemäß Anspruch 16, **gekennzeichnet dadurch, dass** der Schmierstoff ausgewählt ist aus Lösungen von Triglyceriden, von Polyethylenglycol, von Hyaluronat, von Hyaluronsäure, von oxidierter Cellulose, oder von Polysacchariden oder von Mucopolysacchariden.

18. Zusammensetzung zur Verwendung gemäß einem der vorangehenden Ansprüche, **gekennzeichnet dadurch, dass** die Zusammensetzung ein Vernetzungsmittel umfasst.

19. Zusammensetzung zur Verwendung gemäß Anspruch 18, **gekennzeichnet dadurch, dass** das Vernetzungsmittel ausgewählt ist aus Glutaraldehyd, Dialdehyden und Polyaldehyden, insbesondere mit Periodsäure oxidierten Polysacchariden, einschließlich oxidiertem Dextran, oxidiertem Amidon oder oxidierter Hyaluronsäure.

20. Zusammensetzung zur Verwendung gemäß einem der Ansprüche 1 bis 8, **gekennzeichnet dadurch, dass** sie einen Film aus Globin umfasst oder daraus besteht, wobei die Zusammensetzung optional einen Filmbildner enthalten kann, insbesondere einen wie Collagen, Gelatine, Hyaluronsäure, oxidierte Cellulose, Polyethylenglycol, Glycerin.

21. Zusammensetzung zur Verwendung gemäß Anspruch 20, **gekennzeichnet dadurch, dass** der Film durch Dehydratation eines Gels oder einer Lösung erhalten wird.

22. Zusammensetzung zur Verwendung gemäß einem der Ansprüche 1 bis 8, **gekennzeichnet dadurch, dass** sie in Form eines festen Implantats gebildet ist.

23. Zusammensetzung zur Verwendung gemäß einem der Ansprüche 20 bis 22, **gekennzeichnet dadurch, dass** sie vernetzt ist.

24. Zusammensetzung zur Verwendung gemäß Anspruch 23, **gekennzeichnet dadurch, dass** sie durch Zugabe eines Vernetzungsmittels und/oder durch Bestrahlung vernetzt ist.

25. Zusammensetzung zur Verwendung gemäß einem der vorangehenden Ansprüche, **gekennzeichnet dadurch, dass** sie, unter anderem, mindestens einen der folgenden Wirkstoffe enthält: Wundheilmittel, Wachstumsfaktor, Antibiotikum.

26. Zusammensetzung zur Verwendung gemäß einem der vorangehenden Ansprüche, **gekennzeichnet dadurch, dass** sie Zellen enthält, insbesondere Zellen, die, vor Injektion oder Implantierung, unter Verwendung von Globin aus der Zusammensetzung als Kulturmedium, kultiviert wurden, wobei die Zellen insbesondere Hautfibroblasten, Chondrozyten, Osteoblasten oder Stammzellen sein können.

## Claims

1. A preparation in solid or paste form that can be injected or implanted into the human or animal body, which comprises, as main active component, globin that is insoluble at physiological pH, biocompatible and sterile, for use as a tissue filling material.

2. The preparation for use according to claim 1, for filling skin cavities, wrinkles or scars, and bone or cartilage cavities and fractures.

3. A preparation in solid or paste form that can be injected or implanted into the human or animal body, which comprises, as main active component, globin that is insoluble at physiological pH, biocompatible and sterile, for use in tissue volume augmentation.

4. The preparation for use according to claim 1, **characterized in that** it is provided for the augmentation of sphincters, in particular urinary or digestive sphincters, or of vocal cords.

5. A preparation in solid or paste form that can be injected or implanted into the human or animal body, which comprises, as main active component, globin that is insoluble at physiological pH, biocompatible and sterile, in the form of a film and/or compresse, for use for the protection and/or separation of surgical or non surgical external or internal wounds or scars.

6. A preparation in solid or paste form that can be injected or implanted into the human or animal body, which comprises, as main active component, globin that is insoluble at physiological pH, biocompatible and sterile, for use for forming a haemostatic plug for percutaneous arterial wounds, or a skin healing paste, or a cartilage or bone healing material.

7. The preparation for use according to any one of the preceding claims, **characterized in that** the globin is a globin of human origin.

8. The preparation for use according to any one of the preceding claims, **characterized in that** the globin in the preparation is in the precipitated state.

9. The preparation for use according to any one of the preceding claims, **characterized in that** it comprises homogenized globin.

10. The preparation for use according to claim 9, **characterized in that** the globin is in the form of an injectable homogenized paste.

11. The preparation for use according to one of claims 9 and 10, **characterized in that** the homogenized paste can be injected through a hypodermic needle.

12. The preparation for use according to one of claims 9 to 11, **characterized in that** the concentration of globin in the injectable preparation is between 30 and 150 mg/g.

13. The preparation for use according to one of claims 9 to 12, **characterized in that** the pH of the preparation is between 6 and 8.

14. The preparation for use according to one of the preceding claims, **characterized in that** the globin is in suspension.

15. The preparation for use according to one of claims 1 to 8, **characterized in that** the globin is present in the preparation in the form of a gel.

16. The preparation for use according to any one of the preceding claims, **characterized in that** it also comprises a lubricant.

17. The preparation for use according to claim 16, **characterized in that** this lubricant is chosen from solutions of triglycerides, of polyethylene glycol, of hyaluronate, of hyaluronic acid, of oxidized cellulose, or of polysaccharides or of mucopolysaccharides.

18. The preparation for use according to any one of the preceding claims, **characterized in that** the preparation comprises a crosslinking agent.

19. The preparation for use according to claim 18, **characterized in that** the crosslinking agent is chosen from glutaraldehyde, dialdehydes and polyaldehydes, in particular polysaccharides oxidized with periodic acid, including oxidized dextran, oxidized starch or oxidized hyaluronic acid.

20. The preparation for use according to one of claims 1 to 8, **characterized in that** it comprises or consists of a globin film, it being possible for the preparation to optionally contain a film-forming agent, in particular such as collagen, gelatin, hyaluronic acid, oxidized cellulose, polyethylene glycol or glycerol.

21. The preparation for use according to claim 20, **characterized in that** the film has been obtained by dehydration of a gel or of a solution.

22. The preparation for use according to one of claims 1 to 8, **characterized in that** it is produced in the form of a solid implant.

23. The preparation for use according to one of claims 20 to 22, **characterized in that** it is cross-linked.

24. The preparation for use according to claim 23, **characterized in that** it is cross-linked by the addition of a crosslinking agent and/or by irradiation.

25. The preparation for use according to any one of the preceding claims, **characterized in that** it also contains at least one of the following active principles: healing product, growth factor, antibiotic.

26. The preparation for use according to any one of the preceding claims, **characterized in that** it contains cells, in particular cells cultured using the globin of the preparation as culture support, before injection or implantation, which cells can in particular be skin fibroblasts, chondrocytes, osteoblasts or stem cells.
